# EUROPEAN PATENT APPLICATION

(11) **EP 1 260 225 A1**
(43) Date of publication of application: **27.11.2002**
(21) Application number: 01201896.6
(22) Date of filing: 18.05.2001
(51) Int. Cl.: A61K 31/565, A61P 5/30

(54) **A pharmaceutical composition for use in hormone replacement therapy**

(71) Applicant: Pantarhei Bioscience B.V., 3700 AL Zeist (NL)
(72) Inventor: Holinka, Christian Franz, New York, NY 10014 (US); Coelingh Bennink, Herman Jan Tijmen, 3971 BE Driebergen (NL)
(74) Representative: Jorritsma, Ruurd

(57) **Abstract**

The present invention is concerned with a pharmaceutical composition for use in a method of hormone replacement. More particularly, the present invention relates to a pharmaceutical composition for use in a method of hormone replacement therapy, which method comprises administering to a person in need of such a therapy an effective amount of an active component selected from the group consisting of:
substances represented by the following formula 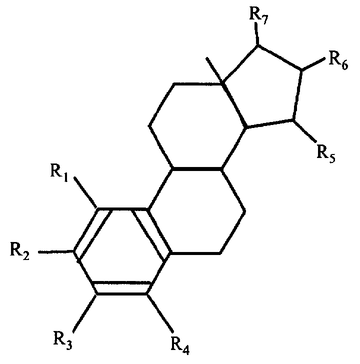 in which formula R₁, R₂, R₃, R₄ independently are a hydrogen atom, a hydroxyl group or an alkoxy group with 1-5 carbon atoms; R₅, R₆, R₇ independently are a hydrogen atom or a hydroxyl group; no more than 3 of R₁, R₂, R₃, R₄, R₅, R_{6,} R₇ are hydrogen atoms and at least 2 of R₅, R_{6,} R₇ are a hydroxyl group;
precursors capable of liberating a substance according to the aforementioned formula when used in the present method, and mixtures thereof;
said composition containing virtually no progestogen or anti-progestin.

An example of a known substance which may suitably be used in the method of the invention is 1,3,5 (10)-estatrien-3, 15α,16α,17β-tetrol (estetrol).

## Description

The present invention is concerned with a pharmaceutical composition for use in a method of hormone replacement. More particularly, the present invention is concerned with the use in such methods of pharmaceutical compositions containing an effective amount of estrogen.

Hormone replacement therapy, sometimes also referred to as estrogen replacement therapy, is commonly applied to prevent or treat the consequences of estrogen deficiency or hypoestrogenism, which may otherwise lead to disorders and ailments such as (post-menopausal) osteoporosis and atherosclerosis, climacteric symptoms such as hot flushes (flashes) and sweats, urogenital atrophy, mood disturbances, insomnia, palpitations and other symptoms related to hypoestrogenism. Hypoestrogenism, and in particular chronic hypoestrogenism, is normally associated with (peri-)menopausal and post-menopausal women. However, if can also result from hypogonadism, castration, primary ovarian failure and gonadotropin-releasing hormone analogue treatment of e.g.endometriosis.

It is well known in the art to treat chronic hypoestrogenism by hormone replacement therapy, i.e. continuous administration of effective amounts of an estrogen for prolonged periods of time. Estrogen replacement therapy has been associated, however, with endometrial proliferation in women and it is now widely accepted that "unopposed" estrogen therapy substantially increases the risk of endometrial cancer (Cushing et al., 1998. Obstet. Gynecol.91, 35-39; Tavani et al., 1999. Drugs Aging, 14, 347-357). There is also evidence of a significant increase in breast cancer with long-term (10-15 years) use of estrogen therapy (Tavani et al., 1999. Drugs Aging, 14, 347-357; Pike et al., 2000. Steroids, 65, 659-664).

In order to counteract the negative effects of unopposed estrogen therapy, adjunctive progestogen treatment is nowadays commonly applied. Regular progestogen administration is believed to inhibit the continual estrogen stimulation of the endometrium through an antiproliferative effect and appears to reduce the incidence of endometrial carcinoma in post-menopausal women receiving estrogen replacement therapy (Beral et al., 1999. J. Epidemiol. Biostat., 4, 191-210). Such an adjunctive treatment, generally using synthetic progestogens, is given either in continuous combined regimens with estrogen, or added sequentially, typically for about 14 days each month, to continuous estrogen treatment. Sequential regimens are associated with undesirable vaginal bleeding in response to periodic progestogen withdrawal, while continuous combined therapy frequently results in unscheduled breakthrough bleeding. Unacceptable bleeding of this type is the most frequent reason for discontinuation of hormone replacement therapy.

Additional adverse effects of adjunctive progestogens include edema, abdominal cramps, breast tenderness and mood symptoms (Hahn, 1989. Am. J. Obstet. Gynecol., 161, 1854-1858). Some synthetic progestogens unfavourably affect lipid patterns and thus negate or attenuate the estrogen induced cardioprotective effects (Sitruk-Ware, 2000. Steroids, 65, 651-658).

To avoid the need for adjunctive progestogens, efforts have been made to synthesise molecules with selective estrogenic properties so as to obtain desirable effects in certain target tissues, such as bone, liver, brain and in tissues that mediate cardioprotection, while minimally affecting other tissues, such as endometrium and breast. These efforts were based on the emerging knowledge that the interaction of estrogens with protein regulating estrogen responsiveness, i.e. estrogen receptors, may differ in different target tissues. Estrogenic compounds, which selectively modulate estrogen receptors in different target tissues and therefore may be expected to have selective estrogen agonistic/antagonistic effects, are known as selective estrogen receptor modulators (SERMs) (Katzenellenbogen et al., 2000. J. Steroid Biochem. Mol. Biol., 74, 279-285; Burger, 2000. Horm. Res., suppl 3, 25-29).

The optimal SERM is expected to affect differentiated cellular functions without inducing proliferation, because enhanced proliferation is associated with increased cancer risk. Regarding the endometrium, exaggerated proliferation (hyperplasia) has been demonstrated to be a precursor of endometrial cancer. Furthermore, the optimal SERM would eliminate the need for adjunctive progestogen treatment and its adverse effects. An example of a synthetic SERM that is commercially available is raloxifene, a benzothiophene anti-estrogen (Clemett et al., 2000. Drugs, 60, 379-411).

It is the objective of the present invention to provide a pharmaceutical composition for use in hormone replacement therapy, wherein said composition contains an effective amount of a substance with SERM-like properties. It has been found that a specific group of estrogens exhibit these highly desirable properties. These estrogens are different from the estrogens commonly applied in estrogen replacement therapy, i.e. ethinyl estradiol, estradiol and its esters such as the acetate, valerate or benzoate, mestranol, the conjugated equine estrogens and estrone sulfate. Thus the present invention is concerned with a pharmaceutical composition for use in a method of hormone replacement therapy, which method comprises administering to a person in need of such a therapy an effective amount of an active component selected from the group consisting of:
substances represented by the following formula 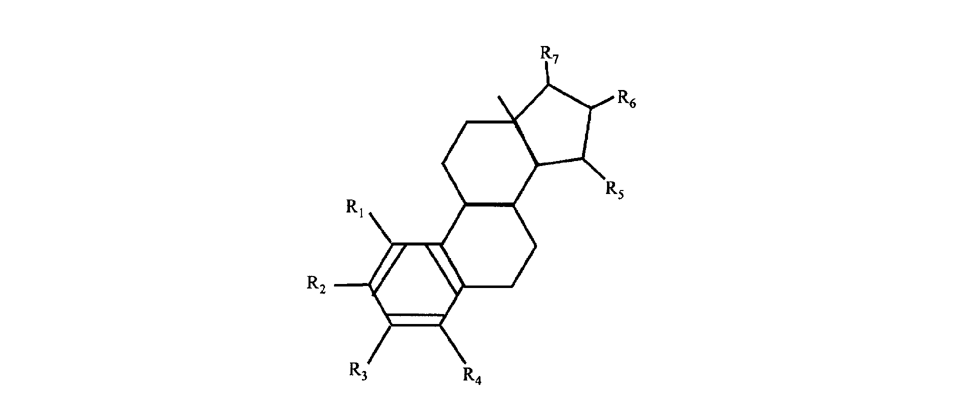 in which formula R₁, R₂, R₃, R₄ independently are a hydrogen atom, a hydroxyl group or an alkoxy group with 1-5 carbon atoms; R₅, R_{6,} R₇ independently are a hydrogen atom or a hydroxyl group; no more than 3 of R₁, R₂, R₃, R₄, R₅, R_{6,} R₇ are hydrogen atoms and at least 2 of R₅, R_{6,} R₇ are a hydroxyl group;
precursors capable of liberating a substance according to the aforementioned formula when used in the present method, and mixtures thereof;
said composition containing virtually no progestogen or anti-progestin.

An example of a substance which is encompassed by the above formula is 1,3,5 (10)-estatrien-3, 15α,16α,17β-tetrol, also known by the name of estetrol. Estetrol is an estrogen that is produced by the fetal liver during human pregnancy. Unconjugated estetrol levels in maternal plasma peak at about 1.2 ng/ml at term pregnancy and are about 12 times higher in fetal than in maternal plasma (Tulchinsky et al., 1975. J. Clin. Endocrinol. Metab., 40, 560-567).

US 5,468,736 (Hodgen) describes a method of hormone replacement therapy involving the administration of estrogen together with an amount of antiprogestin, or antiprogestogen, which inhibits estrogen-induced endometrial proliferation in women. In Example 3 the combined use of estetrol and lilopristone is mentioned. A disadvantage associated with the use of antiprogestogens, such as lilopristone, is the risk of inducing abnormal endometrial morphology, i.e. cystic hyperplasia, as has been observed in women who received an antiprogestogen treatment against endometriosis (Murphy et al., 1995. Fertil. Steril., 95, 761-766).

US 5,340, 586 (Pike et al.) is concerned with compositions and methods which are effective to treat oophorectomized women, wherein an effective amount of an estrogenic composition and an androgenic composition are provided over a period of time. Estetrol is mentioned in a list of estrogens which may be used in these compositions and methods.

Holinka et al., Biol. Reprod. 22, 913-926 (1980) describe the result of a comparison of the effects of estetrol and tamoxifen (an anti-estrogen) with those of estriol and estradiol on the immature rat uterus. It is concluded that estetrol and tamoxifen have estrogenic effects on the immature rat uterus. However, the estrogenic potency of estetrol, relative to estradiol and estriol, was found to be low at the dosage and timing of administration used in the experiments.

Although applicants do not wish to be bound by theory, it is believed that the active component in the pharmaceutical composition according to the present invention interacts with the estrogen receptor in human endometrium in a manner which is distinctly different from the interaction observed for those estrogens that are commonly used in estrogen replacement therapy. It is postulated that estetrol functions as a SERM by selectively interacting with the estrogen receptor to achieve estrogenic effects while minimising the classical growth-promoting effects of the aforementioned commonly used estrogens. In comparison to, for instance, estradiol and ethinyl estradiol, the active components according to the present invention have little or no proliferative effect. Thus, unlike what is the case for these other estrogens, there is no need for adjunctive progestogen or anti-progestogen treatment. The administration of the active components according to the invention without supplementary progestogen or anti-progestogen delivers the full benefits of estrogen replacement therapy without the drawbacks associated with the use of the latter hormones. The composition according to the present invention comprises one or more estrogens or their precursors, which estrogens are distinct from the estrogens commonly applied in estrogen replacement therapy in that they contain at least 4 hydroxyl groups. Examples of commercially available estrogens that contain at least 4-hydroxyl groups and their precursors are:
1, 3, 5(10)-estratrien-2, 3, 15α, 16α, 17β- pentol 2-methyl ether
1, 3, 5(10)-estratrien-2, 3, 15β, 16α, 17β- pentol 2-methyl ether
1, 3, 5(10)-estratrien-2, 3, 16α, 17β- tetrol
1, 3, 5(10)-estratrien-3, 4, 16α, 17β- tetrol 4-methyl ether
1, 3, 5(10)-estratrien-3, 15α, 16α, 17β- tetrol
1, 3, 5(10)-estratrien-3, 15α, 16α, 17β- tetrol
1, 3, 5(10)-estratrien-3, 15α, 16α, 17β- tetrol tetra acetate
1, 3, 5(10)-estratrien-3, 15β, 16β, 17β- tetrol tetra acetate

Preferably, the estrogen substances applied as active component in the present composition are so called biogenic estrogens, i.e. estrogens that naturally occur in the human body. In another preferred embodiment of the present invention the composition contains an active component wherein the estrogen substance contains 4 hydroxyl groups. In yet another preferred embodiment, in the aforementioned formula, at least 2, preferably at least 3 of the groups R₁, R₂, R₃ and R₄ represent a hydrogen atom. Most preferably the groups R₁, R₂ and R₄ represent hydrogen atoms, in which case, if R₃, R₅, R₆ and R₇ are hydroxyl groups, the substance is 1,3,5 (10)-estatrien-3, 15,16,17-tetrol. A preferred isomer of the latter substance is 1,3,5 (10)-estatrien-3, 15α,16α,17β-tetrol (estetrol).

The method of hormone replacement therapy in accordance with the present invention encompasses the treatment, including prophylactic treatment, of ailments, disorders and diseases that can result from estrogen deficiency. In a preferred embodiment the purpose of the present method of hormone replacement therapy is to prevent or treat (peri-)menopausal disorders, post-menopausal disorders, and/or hypoestrogonism resulting from hypogonadism, castration, primary ovarian failure, gonadotropin-releasing hormone analogue treatment of e.g. endometriosis, or estrogen deficiency resulting from cancer treatment. More preferably the purpose is to prevent or treat (peri-)menopausal and post-menopausal disorders. Disorders of the latter type which can advantageously be (prophylactically) treated with the present method include osteoporosis, atherosclerosis, climacteric symptoms such as hot flushes (flashes) and sweats, urogenital atrophy, mood disturbances, insomnia, palpitations. The compositions according to the invention are particularly advantageous when used in a method of hormone replacement therapy for long term treatment of post-menopausal disorders such as osteoporosis.

The present method of hormone replacement therapy comprises administering to a person in need of such a therapy an effective amount of the active component. The amounts needed to be effective will differ from individual to individual and are determined by factors such as the individual's level of estrogen deficiency, body weight, route of administration and the efficacy of the particular estrogen substance used. In general the method according to the invention will comprise the daily administration of 0.001-1000 mg of the active component. Preferably the method comprises orally or transdermally administering, on a daily basis 0.01-1000 mg, and more preferably 0.1-100 mg of the active component.

The composition according to the invention may be administered in a variety of ways as practised in the pharmaceutical art, including oral, transdermal, subcutaneous, intramuscular, intranasal, pulmonary and vaginal administration. In a preferred embodiment, the present composition is administered orally or transdermally.

In a particularly preferred embodiment of the invention the pharmaceutical composition according to invention is designed for daily administration, i.e. it represents a daily dosage unit. In accordance with this preferred embodiment the daily dosage unit contains from 0.001-1000 mg of the active component. More preferably the amount of active component is within the range of 0.01-1000 mg, most preferably it is in the range of 0.1-100 mg.

As mentioned before, it is an important advantage of the present pharmaceutical composition that it can be used to deliver the benefits of estrogen replacement therapy without the drawback of significant estrogen induced endometrial proliferation, thereby obviating the need for using supplementary hormones, e.g. progestogens and anti-progestogens, to suppress such endometrial proliferation.

Hence, in a preferred embodiment of the invention, the composition contains virtually no progestogens or anti-progestogens. The combined use of estrogens and androgens, and estrogens and gonadotropin-releasing hormone have been reported. In an even more preferred embodiment of the present invention, the composition also contains virtually no androgen or gonadotropin-releasing hormone analogue.

Since the benefits of the present invention can be obtained by using the present substances and their precursors as the sole active ingredients, it is preferred that the composition contains virtually no other sex hormones or sex hormone antagonists other than the active component. More preferably, the total level of sex hormones or sex hormone antagonists other than the active component is less than 1%, and most preferably it is less than 0.1% by weight of the active component. However, it should be understood that the present composition may suitably comprise a variety of other supplementary pharmaceutical active components. In particular, it can be advantageous to additionally include anti-resorptive agents such as calcitonins and bisphosphonates. Also, it may be beneficial to incorporate components that act as anabolic agents, such as fluoride salts, growth hormone, insulin-like growth factors, parathyroid hormone, statins, calcium and vitamin D. Furthermore, it may advantageous to include cardiovascular protective agents, such as statins and folic acid.

The pharmaceutical compositions according to the invention can be solid or semi-solid dosage forms such as tablets, capsules, cachets, pellets, pills, powders and granules, as well as fluid dosage forms such as solutions, emulsions, suspensions, ointments, pastes, creams, gels, jellies and foams.

Tablets and equivalent solid and semi-solid dosage forms can suitably contain materials such binders (e.g. hydroxypropylmethyl cellulose, polyvinyl pyrrolidine, other cellulosic materials and starch), diluents (e.g. lactose and other sugars, starch, dicalcium phosphate and cellulosic materials), disintegrating agents (e.g. starch polymers and cellulosic materials) and lubricating agents (e.g., stearates and talc).

Transdermal delivery systems include patches, gels, tapes and creams, and can contain excipients such as solubilisers, permeation enhancers (e.g. fatty acids, fatty acid esters, fatty alcohols and amino acids), hydrophilic polymers (e.g. polycarbophil and polyvinyl pyrrolidine) and adhesives and tackifiers (e.g. polyisobutylenes, silicone-based adhesives, acrylates and polybutene).

Transmucosal delivery systems include patches, tablets, suppositories, pessaries, gels, and creams, and can contain excipients such as solubilizers and enhancers (e.g. propylene glycol, bile salts and amino acids), and other vehicles (e.g. polyethylene glycol, fatty acid esters and derivatives, and hydrophilic polymers such as hydroxypropylmethyl cellulose and hyaluronic acid).

Injectable delivery systems include solutions, suspensions, gels, microspheres and polymeric injectables, and can comprise excipients such as solubility-altering agents (e.g. ethanol, propylene glycol and sucrose) and polymers (e.g. polycaprylactones, and PLGA's). Implantable systems include rods and discs, and can contain excipients such as PLGA and polycapryl lactone.

Other delivery systems that can be used for administering the pharmaceutical composition of the invention include intranasal delivery systems such as sprays and powders, and sublingual delivery systems.

In order for the present composition to be most effective, it is advantageous to ensure that the serum level of the active component is maintained above a certain minimum concentration and that no major concentration fluctuations occur. Hence, in a preferred embodiment of the invention, the composition contains a slow release formulation of the active component which maintains serum levels of said active component at a minimum of 0.1 ng/mL, more preferably between 0.5 and 50 ng/mL. The slow release formulation can take the form of a transdermal patch, or a composition for topical application which gradually releases the active component over time. Alternatively, the slow release formulation can be a composition for oral administration, wherein the active component has been encapsulated in a matrix that will slowly release said component in the gastro-intestinal tract, or wherein the active component is present as a precursor that will liberate the active estrogen in the gastro-intestinal tract or after absorption.

The benefits of the present invention are most pronounced when the composition is used in longer term hormone replacement therapy so as to minimise the negative effects of chronic hypoestrogenism. Therefore, the method of hormone replacement therapy, preferably, comprises administering the active component for a period of at least 1 month, more preferably of at least 3 months

The invention also encompasses the use of precursors of the estrogen substances that constitute the active component in the present pharmaceutical composition. These precursors are capable of liberating the aforementioned estrogen substances when used in the present hormone replacement method, e.g. as a result of metabolic conversion. Typical examples of precursors which can suitably be used in accordance with the invention are esters that can be obtained by reacting the hydroxyl groups of the estrogen substances with substances that contain one or more carboxy (M⁺⁻OOC-) groups, wherein M⁺ represents a hydrogen or (akali)metal cation. Hence, in a preferred embodiment, the precursors are derivatives of the estrogen substances, wherein the hydrogen atom of at least one of the hydroxyl groups in said formula has been substituted by -CO-R, wherein R is a hydrocarbon radical comprising from 1-25 carbon atoms. Preferably R is hydrogen, or an alkyl, alkenyl or aryl radical comprising from 1-20 carbon atoms.

The composition of the invention is particularly suitable for oral administration in the form of e.g. tablets or capsules. Therefore in another embodiment the present invention is concerned with a kit containing at least 20 dosage units wherein each dosage unit comprises a pharmaceutical composition according to the invention. Preferably each dosage unit comprises an amount of active component that is sufficient for 10-48 hours, meaning that the units are to be administered in the present hormone replacement method at intervals between 10 and 48 hours. The dosage units are preferably tablets or capsules. Here the term tablet is meant to encompass other forms of solid bodies that can suitably be used for oral administration, such as pills, pellets and others.

Yet another embodiment of the present invention relates to the use of an active component selected from the group consisting of:
substances represented by the following formula 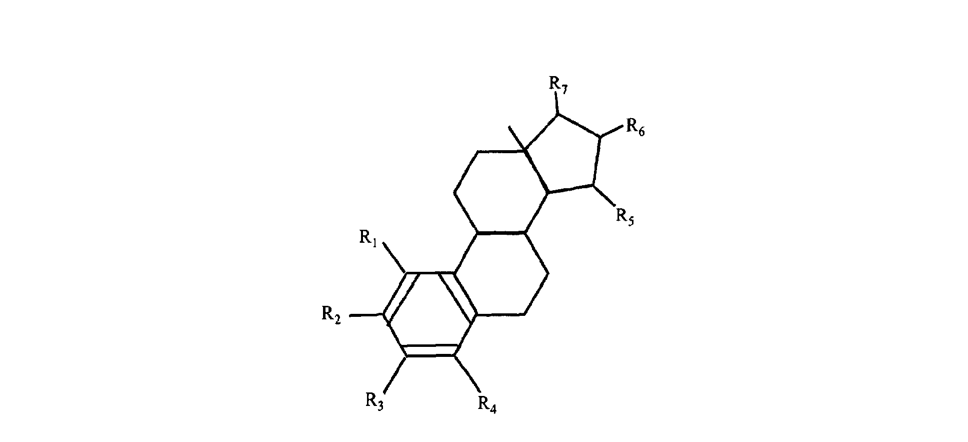 in which formula R₁, R₂, R₃, R₄ independently are a hydrogen atom, a hydroxyl group or an alkoxy group with 1-5 carbon atoms; R₅, R_{6,} R₇ independently are a hydrogen atom or a hydroxyl group; no more than 3 of R₁, R₂, R₃, R₄, R₅, R_{6,} R₇ are hydrogen atoms and at least 2 of R₅, R_{6,} R₇ are a hydroxyl group;
precursors capable of liberating a substance according to the aforementioned formula when used in the present method, and mixtures thereof;
in the preparation of a pharmaceutical composition for use in a method of hormone replacement therapy, which method comprises administering to a person in need of such a therapy an effective amount of the active component, said composition containing virtually no progestogen or anti-progestin

The following example will serve to illustrate the invention:

### Example

The ovariectomized aged rat is used as a model for the human disease osteoporosis. This is an established animal model, recommended by the United States Food and Drug Administration (FDA) (see Guidelines for Pre-clinical and Clinical Evaluation of Agents used in the Treatment or Prevention of Postmenopausel Osteoporosis (1994)) Ovariectomized (OVX-) female Sprague-Dawley rats, age 3 months, are orally dosed (10 per group) with 2.5 mg/kg, 0.5 mg/kg, 0.1 mg/kg per day of estetrol, 0.1 mg/kg per day of 17α-ethynyl-estradiol (EE) or vehicle for 4 weeks.

OVX of rats results in uterine atrophy, increased body weight, changes in cancellous bone turnover, and hypercholesterolemia. EE prevents these changes as expected. Estetrol has similar effects; it prevents the increase in body weight and the increase in blood cholesterol, when administered at 0.1-2.5 mg/kg daily. Moreover, estetrol appears to have no, or only partial, estrogenic activity in the uterus; uterine weight of estetrol-treated animals is significantly less than that of EE-treated animals. Furthermore, estetrol prevents loss of lumbar and proximal tibial bone at doses where no uterotrophic activity is seen in estetrol-treated rats. Correspondingly, in these animals no myometrial and endometrial stimulation is observed at histological evaluation.

We therefore conclude that estetrol has a pharmacologically favourable profile. It lacks agonistic and therefore growth-promoting activity on endometrial tissue. Together with its cholesterol lowering effect and beneficial effects on bone turnover, it has clear therapeutic potential in the treatment of osteoporosis and other hypoestrogenic disorders.

## Claims

1. A pharmaceutical composition for use in a method of hormone replacement therapy, which method comprises administering to a person in need of such a therapy an effective amount of an active component selected from the group consisting of:
substances represented by the following formula 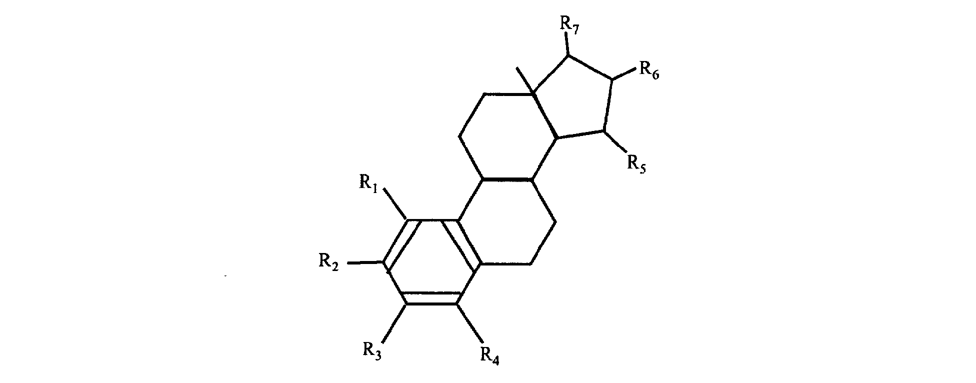 in which formula R₁, R₂, R₃, R₄ independently are a hydrogen atom, a hydroxyl group or an alkoxy group with 1-5 carbon atoms; R₅, R_{6,} R₇ independently are a hydrogen atom or a hydroxyl group; no more than 3 of R₁, R₂, R₃, R₄, R₅, R_{6,} R₇ are hydrogen atoms and at least 2 of R₅, R_{6,} R₇ are a hydroxyl group;
precursors capable of liberating a substance according to the aforementioned formula when used in the present method, and mixtures thereof;
said composition containing virtually no progestogen or anti-progestin.

2. A composition according to claim 1, wherein at least 2, preferably at least 3 of the groups R₁, R₂, R₃ and R₄ represent a hydrogen atom.

3. A composition according to claim 2, wherein groups R₁, R₂ and R₄ represent hydrogen atoms.

4. A composition according to any one of claims 1-3, wherein the purpose of the hormone replacement therapy is to treat (peri-)menopausal disorders, post-menopausal disorders, and/or hypoestrogenism resulting from hypogonadism, castration, primary ovarian failure, gonadotropin-releasing hormone analogue treatment or cancer treatment.

5. A composition according to any one of claims 1-4, wherein the method comprises daily administration of at least 0.001 mg of the active component.

6. A composition according to any one of claims 1-5, wherein the composition is administered orally or transdermally.

7. A composition according to any one of claims 1-6, wherein the composition contains virtually no androgen, or gonadotropin hormone releasing hormone analogue.

8. A composition according to any one of claims 1-7, wherein the composition contains virtually no other sex hormones or sex hormone antagonists other than the active component.

9. A composition according to claim 8, wherein the total level of sex hormones or sex hormone antagonists other than the active component is less than 1% by weight of the active component.

10. A composition according to any one of claims 1-9, wherein the composition contains a slow release formulation of the active component which maintains serum level of said active component at a minimum of 0.1 ng/mL, more preferably between 0.5 and 50 ng/mL.

11. A composition according to any one of claims 1-10, wherein the method comprises administering the active component for a period of at least 1 month, preferably of at least 3 months

12. A composition according to any one of claims 1-11, wherein the precursors are derivatives of the substances represented by the formula of claim 1, wherein the hydrogen atom of at least one of the hydroxyl groups in said formula has been substituted by -CO-R, wherein R is a hydrocarbon radical comprising from 1-25 carbon atoms.

13. A kit containing at least 20 dosage units wherein each dosage unit comprises a pharmaceutical composition according to any one of claims 1-12.

14. Use of an active component selected from the group consisting of:
substances represented by the following formula 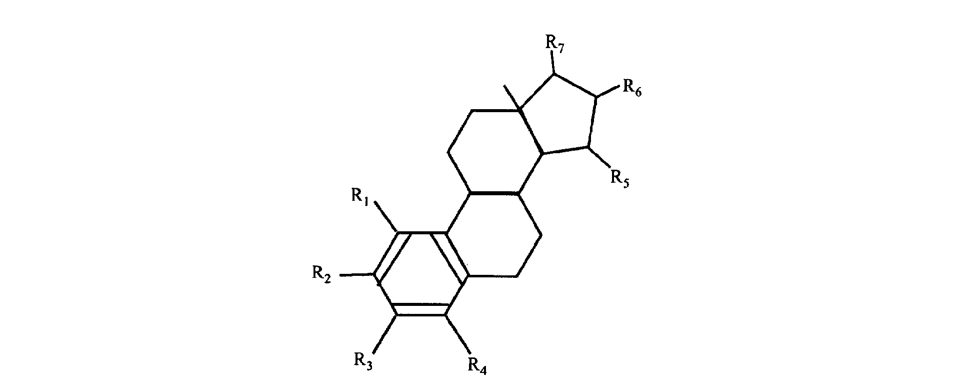 in which formula R₁, R₂, R₃, R₄ independently are a hydrogen atom, a hydroxyl group or an alkoxy group with 1-5 carbon atoms; R₅, R_{6,} R₇ independently are a hydrogen atom or a hydroxyl group; no more than 3 of R₁, R₂, R₃, R₄, R₅, R_{6,} R₇ are hydrogen atoms and at least 2 of R₅, R_{6,} R₇ are a hydroxyl group;
precursors capable of liberating a substance according to the aforementioned formula when used in the present method, and mixtures thereof;
in the preparation of a pharmaceutical composition for use in a method of hormone replacement therapy, which method comprises administering to a person in need of such a therapy an effective amount of the active component, said composition containing virtually no progestogen or anti-progestin.
